# NEW EUROPEAN PATENT SPECIFICATION

(11) **EP 1 978 821 B2**
(45) Date of publication and mention of the opposition decision: **06.02.2019**
(45) Mention of the grant of the patent: 18.01.2012
(21) Application number: 06846849.5
(22) Date of filing: 29.12.2006
(51) Int. Cl.: A23K 10/40, A23L 33/00, A23L 3/10, A23L 33/15, A23L 33/21

(54) **METHOD FOR MODIFYING GUT FLORA IN ANIMALS**
VERFAHREN ZUM MODIFIZIEREN DER DARMFLORA BEI TIEREN
METHODE PERMETTANT DE MODIFIER LA FLORE INTESTINALE CHEZ DES ANIMAUX

(30) Priority: 29.12.2005 US 754807 P
(43) Date of publication of application: 15.10.2008
(73) Proprietor: Hill's Pet Nutrition, Inc., Topeka, KS 66603 (US)
(72) Inventor: KHOO, Christina, Lawrence, KS 66049 (US)
(74) Representative: Wichmann, Hendrik
(86) International application number: PCT/US2006/062702
(87) International publication number: WO 2007/076534

(56) References cited:
- EP-A1- 0 965 344
- WO-A-03/071883
- WO-A1-00/44375
- WO-A1-2005/032545
- WO-A2-2004/000045
- WO-A2-2005/072113
- US-A- 5 780 451
- US-A- 6 156 355
- US-A1- 2004 005 305
- US-A1- 2005 124 576
- US-A1- 2005 124 576

## Description

### Field of the Invention

The present invention relates to methods for modifying the bacterial flora of the gastrointestinal tract (gut), and in some cases reducing gastrointestinal inflammation, in animals, more particularly animals having inflammatory bowel disease (IBD) or at risk for IBD. In certain embodiments the methods involve dietary intervention; accordingly the invention further relates to food compositions useful in practicing such methods.

### Description of the Related Art

IBD has been associated with changes in gut flora; however, as concluded in a review paper by Marteau et al. (2004), Aliment. Pharmacol. Ther. 20(Suppl. 4):18-23, this is a difficult subject and there is need for more research. Various studies reviewed in the Marteau paper have indicated effects on IBD of modifying bacterial flora by administration of probiotics.

Kruis (2004), Aliment. Pharmacol. Ther, 20(Suppl. 4):75-78 reviewed clinical studies of therapeutic effects of probiotics in IBD. It was reported that some studies showed convincing therapeutic benefit, but others did not.

In another review paper by Jergens (1999), Veterinary Clinics of North America: Small Animal Practice 29(2):501-521, it was stated that "evidence of a role for bacterial microflora in canine/feline IBD is presently lacking." Jergens documented dietary therapy for IBD. focusing on hypoallergenic diets, adjusted omega-6 to omega-3 fatty acid ratio and fiber supplementation.

Femandez-Banares et al. (1989). Amer. J. Gastroenterol. 84(7):744-748 reported that human patients with IBD had lower blood plasma levels of certain vitamins, including vitamin C and β-carotene, but not vitamin E, than healthy controls.

Buffinton & Doe (1995), Free Radical Biology & Medicine 19(6):911-918 reported decreased antioxidant defenses in patients with IBD and noted that the anti-inflammatory drug 5-aminosalicylic acid (5-ASA), extensively used in IBD therapy, for example in the form of its prodrug sulfasalazine, has potent antioxidant capacity. Preliminary trials of antioxidant therapy were cited and said to "encourage further exploration of antioxidant strategies for the treatment of IBD."

Lih-Brody et al. (1996). Digestive Diseases & Sciences 41(10):2078-2086 reported a study of reactive oxygen intermediates (ROIs) in the colonic mucosa of IBD patients, and stated that an imbalance in formation of ROIs and antioxidant micronutrients "may provide a rationale for therapeutic modulation with antioxidants."

D'Odorico et al. (2001). Scand, J. Gastroenterol. 36(12):1289-1294 reported reduced antioxidant (including vitamin E and β-carotene) concentration in plasma of IBD patients by comparison with controls and concluded that antioxidant depletion is likely to be important in the pathophysiology of IBD.

González et al. (2001), Int. J. Vitam. Nutr. Res. 71(4):243-250 reported that vitamin E supplementation in a rat model protected the colon from oxidative stress associated with inflammation.

There remains a need in the art for new methods of modifying gut flora, especially adjusting the balance of beneficial and deleterious (e.g., pathogenic) bacteria in the gut in favor of beneficial species, in animals having IBD or at risk for IBD.

### SUMMARY OF THE INVENTION

There is now provided a method for enhancing the balance of beneficial and deleterious bacteria in the gastrointestinal tract of an animal having or at risk for IBD comprising administering to the animal a composition comprising at least one antioxidant, for example vitamin E, vitamin C and/or a carotenoid.

In some embodiments, the enhancement attributable to the method is associated with reduction of inflammation.

Optionally, the method further comprises administering to the animal at least one of a probiotic and a prebiotic.

The invention also provides use of at least one antioxidant in the preparation of a composition for enhancing the balance of beneficial and deleterious bacteria in the gastrointestinal tract of an animal having or at risk for IBD.

### DETAILED DESCRIPTION OF THE INVENTION

A method of the invention is for enhancing the balance of beneficial and deleterious bacteria in the gastrointestinal tract of an animal having or at risk for IBD. The method comprises administering to the animal a composition comprising at least one antioxidant.

The gut flora, i.e., the community of bacteria resident in the gastrointestinal tract, comprises both beneficial and deleterious bacterial types or species. Whether a particular member of the gut flora is beneficial, deleterious or inconsequential to the health of the animal in particular circumstances can depend on a number of factors, but for the purposes of the present invention certain types or species of bacteria can be considered beneficial and others deleterious. Examples of beneficial members of the gut flora include bifidobacteria (species of the genus Bifidobacterium) and lactic acid bacteria, more particularly species of the genus Lactobacillus. Deleterious bacteria include pathogenic bacteria. Examples of deleterious members of the gut flora include Clostridium spp.. Desulfovibrio spp. (including without limitation D. desulfuricans, D. intestinalis and D. vulgaris). Helicobacter spp. (including without limitation H. bizzozeronii, H. felis, H. heilmannii. H. pylori and H. salomonis) and pathogenic forms of Escherichia coli.

Gastrointestinal health typically depends on maintenance of an appropriate balance of beneficial and deleterious bacteria. An increase in the population of deleterious bacteria and/or a decrease in the population of beneficial bacteria can be associated with a decline in gastrointestinal health. Conversely, an increase in the population of beneficial bacteria and/or a decrease in the population of deleterious bacteria can be associated with an improvement in gastrointestinal health, for example restoration of health or remission in an animal having a gastrointestinal disorder such as IBD, or prevention of onset of disease in an animal at risk for such a disorder. The term "associated with" in the present context does not necessarily imply a causal relationship, thus the balance of beneficial and deleterious bacteria can be implicated in pathogenesis or can be merely symptomatic of a gastrointestinal disorder.

"Enhancing" or "enhancement" of the balance herein means shifting the balance in favor of beneficial bacteria, and thus can involve an increase in beneficial bacteria and/or a decrease in deleterious bacteria. In some embodiments of the invention, enhancement of the balance arises from both a reduction in deleterious, e.g., pathogenic, bacteria and an increase in beneficial bacteria.

Bacterial populations in the gut flora can be estimated by any procedure known in the art. For example, stool samples can be cultured using traditional plating methodologies, or illustratively by the fluorescence in situ hybridization (FISH) technique.

The subject animal according to the present method is one "having or at risk for IBD." An animal having IBD is an animal in which any one of a spectrum of inflammatory, gastrointestinal diseases and disorders recognized as a form of IBD has been, professionally diagnosed or an animal exhibiting symptoms consistent with such diagnosis. Such diseases and disorders include without limitation irritable bowel syndrome (IBS), ulcerative colitis and Crohn's disease. An animal having chronic IBD but in remission at the time of application of the method is considered herein to be an animal "having IBD." An animal at risk for IBD is an animal not having a history of IBD or exhibiting IBD symptoms but having one or more risk factors indicating a susceptibility to development of IBD. Such risk factors can include genetic factors (e.g., a family history of IBD) and physiological factors (e.g., elevated levels of one or more pro-inflammatory biomarkers and/or depressed levels of one or more anti-inflammatory biomarkers).

The animal can be human or non-human, including avian, bovine, canine, equine, feline, hircine, murine, ovine and porcine animals. In some embodiments, the animal is a companion animal such as a canine or feline, particularly a dog or a cat.

According to the present method, a composition comprising at least one antioxidant is administered to the animal. Administration can be by any suitable route, including oral or parenteral, but is typically oral. In some embodiments, the composition is a food that is orally administered. Such a food comprising at least one antioxidant can represent a substantial part of the diet of an animal. For example, at least one antioxidant can be present in a food or range of foods consumed daily by the animal and providing substantially complete nutrition of the animal. Alternatively or in addition, at least one antioxidant can be administered as a nutritional supplement, or can be present in a snack, a treat or an at least partially edible toy given to the animal separately from its regular food.

Any nutritionally acceptable antioxidant can be used. In some embodiments, an antioxidant other than one having pharmacological properties (as in the case of drugs such as 5-ASA or prodrugs thereof) is used. In some embodiments, an antioxidant other than a polyphenol is used, although a polyphenol antioxidant such as taxifolin can optionally be present in addition to a non-polyphenol antioxidant. A variety of materials that exhibit free radical quenching or absorbing capacity can be used as antioxidants (for example, fruits, vegetables, certain vitamins, and other chemical compounds). Raw ingredients with high oxygen radical absorbing content include, for example, raw spinach pomace, raw tomato pomace, raw citrus pulp, raw grape pomace, raw carrot granules, raw broccoli, raw green tea, raw corn gluten meal, and raw rice bran. Foods or food products that exhibit free radical quenching or absorbing capacity include, for example, spinach (e.g., spinach pomace), tomato (e.g.. tomato pomace), citrus fruit (e.g.. citrus pulp), grape (e.g., grape pomace), carrot (e.g.. carrot granules), broccoli, corn gluten meal, and rice bran. Compounds that exhibit free radical quenching or absorbing capacity include, for example, vitamin E. vitamin C, carotenoids, coenzyme Q₁₀ (ubiquinone), glutathione, L-carnitine, α-lipoic acid, N-acetylcysteine, S-adenosylmethionine, soy isoflavones and taurine.

In some embodiments, the composition to be administered comprises one or more of vitamin E. vitamin C and a carotenoid.

The term "vitamin E" herein includes any form of vitamin E suitable for consumption by an animal including, but not limited to, any tocopherol or tocotrienol compound, any enantiomer or racemate thereof, and any mixture of such compounds having vitamin E activity. e.g., α-tocopherol (5.7,8-trimethyltocol), β-tocopherol (5,8-dimethyltocol), γ-tocopherol (7.8-dimethyltocol). δ-tocopherol (8-methyltocol). α-tocotrienol (5,7,8-trimethyltocotrienol), β-tocotrienol (5,8-dimethyltocotrienol). γ-tocotrienol (7,8-dimethyltocotrienyl), and δ-tocotrienol (8-methyltocotrienol). Vitamin E can be administered as any one or a mixture of the above compounds or in the form of various derivatives thereof such as esters, including vitamin E acetate, succinate, palmitate and the like, that exhibit vitamin E activity after ingestion by an animal. Typically, vitamin E as used in the present method comprises α-tocopherol or an ester thereof. Vitamin E amounts can be expressed in international units (IU) wherein 1 IU is an amount of vitamin E having vitamin E activity equivalent to 1 mg DL-α-tocopheryl acetate. Alternatively, vitamin E amounts can be expressed as total tocopherol.

The term "vitamin C" herein includes any form of vitamin C suitable for consumption by an animal including, but not limited to, ascorbic acid. L-ascorbic acid, and various derivatives thereof such as calcium phosphate salt, cholesteryl salt, and ascorbate-2-monophosphate. Salts of vitamin C include the sodium salt, calcium salt, zinc salt, and ferrous salt. Esters include stearate, palmitate and like derivatives. Vitamin C can be in any physical form such as a liquid, a semisolid, a solid, or a heat stable form that exhibits vitamin C activity after ingestion by a patient. Vitamin C amounts are expressed herein as ascorbic acid.

The term "carotenoid" herein includes any form of a carotenoid suitable for consumption by an animal including, but not limited to, natural and synthetic carotenoids derived from orange-yellow pigment in plants, algae, leaves, roots, vegetation, tomato meal, red palm oil, tomato powder, and tomato pomace/pulp. The carotenoid β-carotene is a precursor of vitamin A occurring naturally in plants. The antioxidant activity of carotenoids is generally independent of any vitamin A activity they may possess as a result of metabolism to vitamin A by an animal. In this regard, it is noted that cats in particular are generally unable to convert β-carotene to vitamin A. Other carotenoids include astaxanthine, α-carotene, cryptoxanthin, lutein, lycopene and zeaxanthin.

Suitable amounts of one or more antioxidants for administration herein can be expressed in relation to the amount of food (typically expressed on a dry matter basis) consumed by the animal. Illustratively, the antioxidant-containing composition can be administered in an amount providing one or more of:
a. vitamin E in a total tocopherol amount of about 50 to about 1000 µg, for example about 100 to about 800 µg, or about 200 to about 600 µg;
b. vitamin C in an amount of about 30 to about 400 µg, for example about 50 to about 200 µg, or about 75 to about 150 µg: and
c. carotenoid. e.g.. β-carotene, in an amount of about 0.1 to about 5 µg, for example about 0.2 to about 2 µg, or about 0.5 to about 1.5 µg: per gram of food, on a dry matter basis, consumed.

Where the composition to be administered according to the present method is a food forming a substantial part of an animal's diet, the food should typically comprise, on a dry matter basis, one or more of:
a. vitamin E in a total tocopherol amount of about 50 to about 1000 µg/g, for example about 100 to about 800 µg/g, or about 200 to about 600 µg/g;
b. vitamin C in an amount of about 30 to about 400 µg/g, for example about 50 to about 200 µg/g, or about 75 to about 150 µg/g: and
c. carotenoid. e.g., β-carotene, in an amount of about 0.1 to about 5 µg/g, for example about 0.2 to about 2 µg/g, or about 0.5 to about 1.5 µg/g.

In one embodiment, all three of vitamin E. vitamin C and carotenoid (e.g., β-carotene) are present, illustratively in amounts as indicated above.

Where a composition comprising vitamin E, vitamin C and/or a carotenoid is other than a food forming a substantial part of the animal's diet, it should typically be administered in an amount consistent with that set forth above, in relation to the amount of food consumed by the animal. Typically, in the case of a supplement, a snack, a treat or an at least partially edible toy, the composition contains higher concentrations of antioxidant(s) than set forth above, as the total amount of such compositions consumed is substantially lower than in the case of a food forming a substantial part of the diet.

In accordance with the invention, it has surprisingly been found that administration of a food enriched in vitamin E. vitamin C and β-carotene can enhance the balance of beneficial and deleterious bacteria in an animal's gut flora by comparison with a comparative food having lower levels of these antioxidants. In some cases the enhancement involves both an increase in the population of beneficial bacteria and a decrease in the population of deleterious bacteria.

In some embodiments of the invention, the enhancement of gut flora balance attributable to practice of the method is associated with reduction of inflammation, more particularly reduction of gastrointestinal inflammation, such as inflammation of the colonic mucosa. Reduction of inflammation may be directly observable, for example by colonoscopy, or may be evidenced by a decrease in one or more pro-inflammatory biomarkers and/or an increase in one or more anti-inflammatory biomarkers in a biofluid or tissue of the animal. Examples of pro-inflammatory biomarkers that may be decreased in blood serum include C-reactive protein (CRP), oxidized glutathione (GSSG), alkenals, serum amyloid A (SAA) and tumor necrosis factor alpha (TNF-α, a pro-inflammatory cytokine). Examples of anti-inflammatory biomarkers that may be increased in blood serum include reduced glutathione (GSH), oxygen radical absorbing capacity (ORAC), and the anti-inflammatory cytokines transforming growth factor beta (TGF-β) and interleukin 10 (IL-10).

Optionally, a method of the invention comprises, in addition to administration of a composition comprising an antioxidant, administration of one or more of a probiotic and a prebiotic.

A probiotic is a preparation or composition comprising viable microbes, for example bacteria, molds or yeasts. Probiotics of interest herein comprise at least one kind of beneficial bacteria, for example bifidobacteria and/or lactic acid bacteria. In one embodiment a probiotic useful herein comprises beneficial bacteria comprising one or more of Bifidobacterium spp, and Lactobacillus spp. Suitable species include, without limitation. Bifidobacterium animalis (including B. animalis subsp, lactis, sometimes referred to as B. lactis). Bifidobacterium longum (including B. infantis). Bifidobacterium thermophilum. Lactobacillus acidophilus. Lactobacillus animalis, Lactobacillus casei, Lactobacillus plantarum. Lactobacillus reuteri, and Lactobacillus rhamnosus.

A probiotic can be included in the same composition as the antioxidant, for example in a food composition, or it can be administered separately. A suitable amount of a probiotic is generally about 10⁶ to about 10¹² cfu (colony forming units) per gram of food on a dry matter basis consumed by the animal.

In combination with the antioxidant, a probiotic can enhance the balance of the gut flora simply by acting as an inoculum for an increased population of beneficial bacteria, and/or by antagonizing growth of deleterious bacteria. In some situations co-action of the antioxidant and a probiotic can occur, and this may under some circumstances lead to a synergistic interaction of the antioxidant and the probiotic, although such synergism is not a requirement herein.

A prebiotic is a nondigestible substance that preferentially stimulates growth of beneficial bacteria. Most prebiotics are fermentable carbohydrates; examples include oligosaccharides, galactans and β-glucans, obtainable from various plant and microbial sources. Specific examples include arabinogalactan, fructooligosaccharide (FOS) and inulin a polysaccharide that yields FOS. The prebiotic can be administered separately from or in the same composition, e.g., a food composition, as the antioxidant.

In yet another embodiment, a method of the invention comprises administering an anti-IBD agent, in addition to a composition comprising an antioxidant, and optionally a probiotic and/or a prebiotic. An "anti-IBD agent" herein is a pharmacological agent, typically a drug or herbal preparation, providing therapeutic benefit to an IBD patient, for example in reducing inflammation, alleviating symptoms such as pain and/or diarrhea, etc. Suitable examples include, without limitation, steroids such as beclomethasone, budesonide, prednisolone, prednisone and tixocortol; 5-ASA releasing preparations such as mesalamine, olsalazine and sulfasalazine; metronidazole: azathioprine: etc. The anti-IBD agent can be administered by any suitable route, including oral, parenteral, transdermal and rectal routes. If desired, an anti-IBD agent suitable for oral administration can be included in the same composition as the antioxidant.

Also an aspect of the invention is use of at least one antioxidant in preparation of a composition for enhancing the balance of beneficial and deleterious bacteria in the gastrointestinal tract of an animal having or at risk for IBD. All embodiments described or enumerated above for a method of the invention apply equally to such use.

The invention provides, in yet another embodiment, a food composition, useful for example in practice of a method as described herein. A food composition of the present embodiment typically has a content of metabolizable energy and nutrients that render it suitable as a substantial part of the diet of an animal. The composition comprises, on a dry matter basis. (1) at least one of:
a. vitamin E in a total tocopherol amount of about 50 to about 1000 µg/g, for example about 100 to about 800 µg/g, or about 200 to about 600 µg/g:
b. vitamin C in an amount of about 30 to about 400 µg/g, for example about 50 to about 200 µg/g, or about 75 to about 150 µg/g: and
c. carotenoid. e.g., β-carotene, in an amount of about 0.1 to about 5 µg/g, for example about 0.2 to about 2 µg/g, or about 0.5 to about 1.5 µg/g;
and (2) at least one of a probiotic and a prebiotic.

In an illustrative embodiment, the composition comprises a probiotic comprising beneficial bacteria of one or more of Bifidobacterium spp, and Lactobacillus spp. A suitable amount of probiotic is typically about 10⁶ to about 10¹² cfu/g, on a dry matter basis.

In another illustrative embodiment, the composition comprises a prebiotic comprising one or more of an oligosaccharide, a galactan or a β-glucan.

The food composition generally contains amounts and a balance of nutrients appropriate to the animal for which it is intended. In one embodiment the composition is a pet food, for example a food nutritionally and/or organoleptically adapted for feeding to a companion animal such as a canine or feline.

The invention is not limited to the particular methodology, protocols, and reagents described herein because they may vary. Further, the terminology used herein is for the purpose of describing particular embodiments only and is not intended to limit the scope of the present invention. As used herein and in the appended claims, the singular forms "a," "an," and "the" include plural reference unless the context clearly dictates otherwise. Similarly, the words "comprise". "comprises", and "comprising" are to be interpreted inclusively rather than exclusively.

Unless defined otherwise, all technical and scientific terms and any acronyms used herein have the same meanings as commonly understood by one of ordinary skill in the art in the field of the invention. Although any methods and materials similar or equivalent to those described herein can be used in the practice of the present invention, the preferred methods, devices, and materials are described herein.

All patents, patent applications, and publications mentioned herein are incorporated herein by reference to the extent allowed by law for the purpose of describing and disclosing the compounds, processes, techniques, procedures, technology, articles, and other compositions and methods disclosed therein that might be used with the present invention. However, nothing herein is to be construed as an admission that the invention is not entitled to antedate such disclosure by virtue of prior invention.

### EXAMPLES

The invention can be further illustrated by the following examples of preferred embodiments thereof, although it will be understood that these examples are included merely for purposes of illustration and are not intended to limit the scope of the invention unless otherwise specifically indicated.

### Example 1

A study is conducted with 11 healthy (not having IBD) cats and 11 cats diagnosed with IBD. A standard canned feline pet food with or without antioxidant enrichment (see analysis in Table 1) is fed to the cats for 2 weeks, followed by crossover to the other food, which is fed for a further 2 weeks.

**Table 1**

| Analysis of Non-enriched (control) and Enriched (test) Foods (DM = Dry Matter) | | |
|---|---|---|
| | Control Food | Test Food |
| Moisture (%) | 75 | 74 |
| Protein (%) | 9 | 9 |
| Fat (%) | 8 | 8 |
| Carbohydrate (%) | 6 | 7 |
| Crude Fiber (%) | 0.2 | 0.2 |
| Total Tocopherol (µg/g DM) | 79 | 221 |
| Vitamin C (µg/g DM) | 34 | 87 |
| β-Carotene (µg/g DM) | 0.05 | 0.92 |

At the end of each 2-week feeding period, blood samples are taken from each cat. Serum levels, of reduced and oxidized glutathione (GSH and GSSG respectively) as antioxidant biomarkers are measured. Mean levels are shown in Table 2. Also. T lymphocyte proliferation levels in response to non-specific mitogens concanavalin A (ConA) and pokeweed mitogen (PWM) are measured as biomarkers of inflammatory response. Mean response levels are shown in Table 3.

**Table 2**

| GSH and GSSG Levels in Serum of Cats on Control and Test Foods | | | | |
|---|---|---|---|---|
| | GSH (pmol/g protein) | | GSSG (pmol/g protein) | |
| | Control | Test | Control | Test |
| Healthy Cats | 9.1 | 9.7 | 0.2 | 0.2 |
| Cats with IBD | 8.5 | 9.1 | 0.2 | 0.2 |

**Table 3**

| T Lymphocyte Proliferation Response to Mitogens ConA and PWM | | | | |
|---|---|---|---|---|
| | ConA | | PWM | |
| | Control | Test | Control | Test |
| Healthy Cats | 1.1 | 1.1 | 0.6 | 0.6 |
| Cats with IBD | 1.4 | 1.3 | 0.9 | 0.8 |

As shown in Table 2, feeding the antioxidant-enriched food results in higher GSH levels in both healthy cats and cats with IBD. When fed the control (non-enriched) food, the GSH level in cats with IBD is lower than that in healthy cats; feeding the antioxidant-enriched food raises the GSH level in cats with IBD to a level comparable to that of healthy cats on the control food.

As shown in Table 3, feeding the antioxidant-enriched food to cats with IBD brings T lymphocyte proliferation response to a level closer to that observed in healthy cats. However, this response is not completely normalized (did not become equal to that in healthy cats).

### Example 2

A study is conducted with 11 healthy cats and 11 cats diagnosed with IBD. Each of the same control (non-enriched) and test (antioxidant-enriched) foods as in Example 1. is fed to the cats for 4 weeks, followed by crossover to the other food, which is fed for a further 4 weeks. Stool (fecal) samples are collected at the beginning and end of each 4-week feeding period and are submitted for bacterial culture using traditional plating methodology. Counts for two kinds of deleterious bacteria (clostridia and E. coli) and two kinds of beneficial bacteria (lactic acid bacteria and bifidobacteria) are obtained. Results are shown in Table 4.

**Table 4**

| Log10 cfu/g Feces by Traditional Plating Methodology | | | | | |
|---|---|---|---|---|---|
| | | Clostridia | E. Coli | Lactic Acid Bacteria | Bifidobacteria |
| Healthy Cats | Control | 7.51 | 6.43 | 8.73 | 9.33 |
| | Test | 6.32 | 5.77 | 8.30 | 10.21 |
| Cats with IBD | Control | 7.67 | 9.41 | 8.69 | 9.91 |
| | Test | 7.60 | 8.48 | 8.95 | 10.42 |

As shown in Table 4, E. coli population is about 3 log units (about one thousand times) higher in samples from cats with IBD than in samples from healthy cats. Cats fed the antioxidant-enriched food exhibit a decline of about 0.8 to about I log unit in E. coli population by comparison with the control food. The antioxidant-enriched food also tend to lower Clostridium populations, although this effect is more marked in healthy cats than in cats with IBD. Meanwhile, populations of beneficial bacteria (both lactic acid bacteria and bifidobacteria) are increased in cats with IBD fed the antioxidant-enriched food by comparison with those fed the control food. In healthy cats only the bifidobacteria show an increase in response to the antioxidant-enriched food in this study.

It can be concluded from this study that administration of an antioxidant-enriched diet to animals with IBD can enhance the balance of beneficial and deleterious bacteria in the gut flora. Such enhancement can be further assisted, for example by supplementing an antioxidant-enriched food with one or more probiotics comprising beneficial bacteria such as Lactobacillus spp, or Bifidobacterium spp.

In the specification, there have been disclosed atypical preferred embodiments of the invention and, although specific terms are employed, they are used in a generic and descriptive sense only and not for purposes of limitation, the scope of the invention being set forth in the claims. Obviously many modifications and variations of the invention are possible in light of the above teachings. It is therefore to be understood that within the scope of the appended claims the invention may be practiced otherwise than as specifically described.

## Claims

1. One or more antioxidants for use in enhancing the balance of beneficial and deleterious bacteria in the gastrointestinal tract of an animal having or at risk for inflammatory bowel disease (IBD).

2. The one or more antioxidants of Claim 1 wherein the enhancement comprises an increase in level of beneficial bacteria and a decrease in level of deleterious bacteria.

3. The one or more antioxidants of Claim 1 or Claim 2 wherein the beneficial bacteria comprise one or more of Lactobacillus spp. and Bifidobacterium spp and/or the deleterious bacteria comprise one or more of Clostridium spp., Desulfovibrio spp., Helicobacter spp. and pathogenic forms of Escherichia coli.

4. The one or more antioxidants of any preceding claim wherein the enhancement is associated with reduction of inflammation, and wherein preferably the reduction of inflammation is evidenced by a decrease in a pro-inflammatory biomarker and/or an increase in an anti-inflammatory biomarker in a biofluid or tissue of the animal.

5. The one or more antioxidants of Claim 4 wherein at least one of serum CRP, oxidized glutathione, alkenals, amyloid and TNF-α is decreased and/or at least one of serum reduced glutathione, ORACs, TGF-β and IL-10 is increased.

6. The one or more antioxidants of any preceding claim wherein the antioxidant comprises one or more of vitamin E, vitamin C and a carotenoid.

7. The one or more antioxidants according to any preceding claim which is a food, a supplement, a snack, a treat, or an at least partially edible toy, preferably a food administered orally as a part of the diet of the animal.

8. The one or more antioxidants according to any preceding claim in conjunction with one or more anti-IBD agents as a combined preparation for use in enhancing the balance of beneficial and deleterious bacteria in the gastrointestinal tract of an animal having or at risk for IBD.

9. The one or more antioxidants of any preceding claim wherein the animal is canine or feline.

10. Use of at least one antioxidant in preparation of a composition for enhancing the balance of beneficial and deleterious bacteria in the gastrointestinal tract of an animal having or at risk for IBD.

## Patentansprüche

1. Ein oder mehrere Antioxidantien zur Verwendung bei der Förderung der Ausgewogenheit von nützlichen und schädlichen Bakterien im Magen-Darm-Trakt eines Tiers mit entzündlicher Darmerkrankung (*inflammatory bowel disease,* IBD) oder einem Risiko dafür.

2. Ein oder mehrere Antioxidantien nach Anspruch 1, wobei die Förderung eine Erhöhung des Spiegels an nützlichen Bakterien und eine Senkung des Spiegels von schädlichen Bakterien einschließt.

3. Ein oder mehrere Antioxidantien nach Anspruch 1 oder Anspruch 2, wobei die nützlichen Bakterien ein oder mehrere von Lactobacillus spp. und Bifidobacterium spp umfassen und/oder die schädlichen Bakterien ein oder mehrere von Clostridium spp., Desulfovibrio spp., Helicobacter spp. und pathogene Formen von Escherichia coli umfassen.

4. Ein oder mehrere Antioxidantien nach einem beliebigen vorhergehenden Anspruch, wobei die Förderung mit einem Abklingen von Entzündung einhergeht, und wobei das Abklingen von Entzündung vorzugsweise durch eine Senkung eines proinflammatorischen Biomarkers und/oder eine Erhöhung eines antiinflammatorischen Biomarkers in einem Biofluoid oder Gewebe des Tiers nachweisbar ist.

5. Ein oder mehrere Antioxidantien nach Anspruch 4, wobei mindestens eines von Serum-CRP, oxidiertem Glutathion, Alkenalen, Amyloid und TNF-α gesenkt und/oder mindestens eines von reduziertem Glutathion im Serum, ORACs, TGF-β und IL-10 erhöht ist.

6. Ein oder mehrere Antioxidantien nach einem beliebigen vorhergehenden Anspruch, wobei das Antioxidans eines oder mehrere von Vitamin E, Vitamin C und einem Carotinoid umfasst.

7. Ein oder mehrere Antioxidantien nach einem beliebigen vorhergehenden Anspruch, welches ein Nahrungsmittel, ein Nahrungsergänzungsmittel, eine Zwischenmahlzeit, ein Leckerli oder ein zumindest teilweise essbares Spielzeug ist, vorzugsweise ein Nahrungsmittel, das oral als ein Teil der Ernährung des Tiers verabreicht wird.

8. Ein oder mehrere Antioxidantien nach einem beliebigen vorhergehenden Anspruch zusammen mit einem oder mehreren Anti-IBD-Mitteln als eine kombinierte Zubereitung zur Verwendung bei der Förderung der Ausgewogenheit von nützlichen und schädlichen Bakterien im Magen-Darm-Trakt eines Tiers mit IBD oder einem Risiko dafür.

9. Ein oder mehrere Antioxidantien nach einem beliebigen vorhergehenden Anspruch, wobei das Tier ein Hund oder eine Katze ist.

10. Verwendung von mindestens einem Antioxidans bei der Herstellung einer Zusammensetzung zur Förderung der Ausgewogenheit von nützlichen und schädlichen Bakterien im Magen-Darm-Trakt eines Tiers mit IBD oder einem Risiko dafür.

## Revendications

1. Un ou plusieurs antioxydants pour une utilisation dans l'amélioration de l'équilibre entre les bactéries bénéfiques et délétères dans le tube digestif d'un animal présentant ou risquant de présenter une maladie inflammatoire intestinale (IBD).

2. Un ou plusieurs antioxydants selon la revendication 1, dans lesquels l'amélioration comprend une augmentation du taux de bactéries bénéfiques et une diminution du taux de bactéries délétères.

3. Un ou plusieurs antioxydants selon la revendication 1 ou la revendication 2, dans lesquels les bactéries bénéfiques comprennent une ou plusieurs bactéries parmi Lactobacillus spp. et Bifidobacterium spp. et/ou les bactéries délétères comprennent une ou plusieurs bactéries parmi Clostridium spp., Desulfovibrio spp., Helicobacter spp. et les formes pathogènes d'Escherichia coli.

4. Un ou plusieurs antioxydants selon l'une quelconque des revendications précédentes, dans lesquels l'amélioration est associée à une réduction de l'inflammation, et dans lesquels de préférence la réduction de l'inflammation est mise en évidence par une diminution d'un biomarqueur pro-inflammatoire et/ou une augmentation d'un biomarqueur anti-inflammatoire dans un fluide biologique ou un tissu de l'animal.

5. Un ou plusieurs antioxydants selon la revendication 4, dans lesquels au moins l'un parmi la CRP sérique, le glutathion oxydé, les alcénals, l'amyloïde et le TNF-α est diminué et/ou au moins l'un parmi le glutathion réduit sérique, les ORAC, le TGF-β et l'IL-10 est augmenté.

6. Un ou plusieurs antioxydants selon l'une quelconque des revendications précédentes, dans lesquels l'antioxydant comprend un ou plusieurs parmi la vitamine E, la vitamine C et un caroténoïde.

7. Un ou plusieurs antioxydants selon l'une quelconque des revendications précédentes, qui sont un aliment, un supplément, un encas, une friandise, ou un jouet au moins partiellement comestible, de préférence un aliment administré par voie orale en tant que partie du régime alimentaire de l'animal.

8. Un ou plusieurs antioxydants selon l'une quelconque des revendications précédentes, conjointement à un ou plusieurs agents anti-IBD sous la forme d'une préparation combinée pour une utilisation dans l'amélioration de l'équilibre entre les bactéries bénéfiques et délétères dans le tube digestif d'un animal présentant ou risquant de présenter une IBD.

9. Un ou plusieurs antioxydants selon l'une quelconque des revendications précédentes, dans lesquels l'animal est un canidé ou un félidé.

10. Utilisation d'au moins un antioxydant dans la préparation d'une composition pour améliorer l'équilibre entre les bactéries bénéfiques et délétères dans le tube digestif d'un animal présentant ou risquant de présenter une IBD.
